# EUROPEAN PATENT SPECIFICATION

(11) **EP 1 904 515 B1**
(45) Date of publication and mention of the grant of the patent: **08.12.2010**
(21) Application number: 06754416.3
(22) Date of filing: 16.06.2006
(51) Int. Cl.: C07K 1/04

(54) **SIDE-CHAIN EXTENDED LIGATION**
SEITENKETTENVERLÄNGERNDE LIGIERUNG
LIGATION ETENDUE DE CHAINE LATERALE

(30) Priority: 16.06.2005 US 691396 P
(43) Date of publication of application: 02.04.2008
(73) Proprietor: AMYLIN PHARMACEUTICALS, INC., San Diego, CA 92121 (US)
(72) Inventor: BOTTI, Paolo, CH-1234 Vessy (CH); TCHERTCHIAN, Sylvie, F-74560 Monnetier Mornex (FR)
(74) Representative: UEXKÜLL & STOLBERG
(86) International application number: PCT/EP2006/005815
(87) International publication number: WO 2006/133962

(56) References cited:
- YAN L Z; DAWSON P E: "Synthesis of peptides and proteins without cysteine residues by native chemical ligation combined with desulfurization." JOURNAL OF THE AMERICAN CHEMICAL SOCIET, vol. 123, no. 3, 31 January 2001 (2001-01-31), pages 526-533, XP002393399 cited in the application
- MOSBERG H I; HAASETH R C; RAMALINGAM K; MANSOUR A; AKIL H; WOODARD R W: "Role of steric interactions in the delta opioid receptor selectivity of [D-Pen2, D-Pen5]enkephalin" INTERNATIONAL JOURNAL OF PEPTIDE AND PROTEIN RESEARCH, vol. 32, no. 1, July 1988 (1988-07), pages 1-8, XP008067371
- MOSBERG H I; HURST R; HRUBY V J; GEE K; YAMAMURA H I; GALLIGAN J J; BURKS T F: "Bis-penicillamine enkephalins possess highly improved specificity toward delta opioid receptors." PROCEEDINGS OF THE NATIONAL ACADEMY OF SCIENCES OF THE UNITED STATES OF AMERICA, vol. 80, no. 19, October 1983 (1983-10), pages 5871-5874, XP002393464
- LAGO M A; SAMANEN J; ELLIOTT J D: "Enantioselective routes to protected syn- and anti-[beta]-phenylcysteines" JOURNAL OF ORGANIC CHEMISTRY, vol. 57, no. 12, 5 June 1992 (1992-06-05), pages 3493-3496, XP002393400
- NARAYAN RADHA S; VANNIEUWENHZE MICHAEL S: "Versatile and stereoselective syntheses of orthogonally protected beta-methylcysteine and beta-methyllanthionine" ORGANIC LETTERS, vol. 7, no. 13, 20 May 2005 (2005-05-20), pages 2655-2658, XP002393401
- INGUIMBERT N; CORIC P; DHOTEL H; BONNARD E; LLORENS-CORTES C; MOTA N; FOURNIÉ-ZALUSKI M-C; ROQUES B-P: "Synthesis and in vitro activities of new non-peptidic APA inhibitors" THE JOURNAL OF PEPTIDE RESEARCH, vol. 65, no. 2, February 2005 (2005-02), pages 175-188, XP002393402
- CORSE, JOSEPH; KLEIDERER, E. C.; SOPER, QUENTIN F.: "New Compounds. ?-Amino-?-mercapto-n-valeric Acid Hydrochloride" JOURNAL OF THE AMERICAN CHEMICAL SOCIETY, vol. 70, no. 1, January 1948 (1948-01), pages 438-439, XP002393403

## Description

### CROSS-REFERENCES TO RELATED APPLICATIONS

This application claims priority to U.S. Provisional Patent Application No. 60/691, 396, filed June 16, 2005.

### BACKGROUND OF THE INVENTION

Chemical ligation involves the formation of a covalent linkage between a first chemical component and a second chemical component. Unique, mutually reactive functional groups present on the first and second components can be used to render the ligation reaction chemoselective. Several different chemistries have been utilized for this purpose, examples of which include amide-forming ligation (Dawson et al., Science 1994, 266, 776-779; Kent et al., WO 96/34878; Kent et al., WO 98/28434; Botti et al., WO 02/20557; and Kent et al., WO 02/20034); oxime forming chemical ligation (Rose et al., J. Amer. Chem. Soc. 1994, 116, 30-34), thioester forming ligation (Schnölzer et al., Science 1992, 256, 221-225), thioether forming ligation (Englebretsen et al., Tet. Letts. 1995, 36, 8871-8874), hydrazone forming ligation (Gaertner et al., Bioconj. Chem. 1994, 5, 333-338), thiazolidine forming ligation and oxazolidine forming ligation (Zhang et al., Proc. Natl. Acad. Sci. 1998, 95, 9184-9189; Tam et al., WO 95/00846; US Patent No. ,589,356).

The most widely used amide-forming ligation is referred to as native chemical ligation ("NCL") (Kent et al., WO 96/34878). Native chemical ligation involves a chemoselective reaction between a first peptide or polypeptide segment having a C-terminal α-carboxythioester moiety and a second peptide or polypeptide having an N-terminal cysteine residue. A thiol exchange reaction yields an initial thioester-linked intermediate, which spontaneously rearranges to give a native amide bond at the ligation site while regenerating the cysteine side chain thiol. Native chemical ligation has been adapted for multiple purposes, including synthesis of polypeptides and proteins on a solid phase (Canne et al., WO 98/56807; Low et al., WO 04/105685), synthesis of membrane polypeptides and proteins and general access to difficult peptides (Kochendoerfer et al., WO 00/12536), synthesis of soluble receptor domains (Kochendoerfer, G., WO 00/53624), construction of polymer-modified peptides and proteins (Kochendoerfer et al., WO 02/20033), as well as modified for application with water-soluble protecting groups (Kochendoerfer et al., WO 04/060925), reducing side reactions (Kochendoerfer et al., WO 02/20033; Botti et al., WO 04/007661), expanding protecting group options (Kochendoerfer et al., WO 02/20033; Villain et al., WO 02/098902), and fostering alternative thioester-generating strategies (Botti et al., WO 02/18417; Botti et al., WO 03/106615; Miranda et al., WO 04/061094; Miranda et al., WO 04/011424; Miranda, L., WO 04/060863). The primary drawback of the original NCL technique is a requirement for an N-terminal cysteine, i.e., it only permits the joining of peptides and polypeptide segments possessing a cysteine at the ligation site.

Amide-forming chemical ligation at positions other than cysteine has been reported. One approach, termed extended native chemical ligation or ENCL, employs the adjoining of a first peptide bearing a C-terminal carboxythioester with a second peptide segment bearing an N-terminal amino-substituted auxiliary thiol group that is removable after ligation (Botti et al., WO 02/20557; Botti et al., WO 03/042235). A second approach, termed pseudo-native chemical ligation or PNCL, involves reacting a C-terminal carboxythioester peptide with a peptide segment bearing an N-terminal cysteine (as with NCL), followed by chemical modification of the side chain thiol of the cysteine at the ligation site to generate a 'pseudo-amino acid' (Kent et al., WO 02/20034). A drawback of the ENCL and PNCL is the variable reaction yield related to particular amino acids employed at the ligation site. For example, as with NCL, the ENCL and PNCL approaches work poorly at ligation sites where β-branched amino acids are involved.

Accordingly, what is needed is a broadly applicable and robust chemical ligation system that provides alternatives to previous amide-forming ligation techniques. The present invention addresses these and other needs.

### BRIEF SUMMARY OF THE INVENTION

The present invention relates to methods and compositions for side chain extended chemical ligations. Advantageously, the ligation reactions have fast reaction rates and produce ligation products in high yield.

According to one aspect, the present invention provides a method of ligating components through an amide bond. The method includes contacting a component of the formula J₁-C(O)SR with a component of the formula NH₂-CH(XSH)-J₂ under conditions sufficient to form a thiol-substituted ligation product having the formula J₁-C(O)-NH-CH(XSH)-J₂, where, J₁ is a residue of a first target molecule, polymer or surface; R is a group compatible with thioesters; XSH is a branched amino acid side chain having a removable thiol auxiliary and selected from the group consisting of a β-mercapto auxiliary of the formula -CH(R')-SH and a γ-mercapto auxiliary of the formula -CH₂-CH(R')-SH, wherein R' is selected from the group consisting of ethylene, phenyl, hydroxylphenyl, allylthioalkyl, carbamoyl (-C(O)NH2), carbamoylalkyl, indolyl, hydroxycarbonyl, guanidine alkyl, and imidazolyl; and J₂ is a residue of a second target molecule, polymer or surface. In a further aspect, the present invention also provides a method of removing the thiol auxiliary to generate a ligation product of the formula J₁-C(O)-NH-CH(XH)-J₂, wherein XH is an amino acid side chain having a formula selected from the group consisting of: -CH₂(R') and -CH₂-CH₂(R').

According to another aspect, the present invention provides a component having the formula P₁-NH-CH(XSH)-J₂, where P₁ is a removable amino protecting group or hydrogen; XSH is a branched amino acid side chain component and selected from the group consisting of a β-mercapto auxiliary of the formula -CH(R')-SH and a γ-mercapto auxiliary of the formula -CH₂-CH(R')-SH, where R' is selected from the group consisting of ethylene, phenyl, hydroxylphenyl, allylthioalkyl, carbamoyl (-C(O)NH2), carbamoylalkyl, indolyl, hydroxycarbonyl, guanidine alkyl, and imidazolyl and the residues CH₂R' and CH₂CH₂R' produced after removal of the thiol auxiliary are side chains of amino acids; and J₂ is a residue of a target molecule, polymer or a surface.

According to yet another aspect, the present invention provides a ligation product having the formula J₁-C(O)-NH-CH(XSH)-J₂, where J₁ is a residue of a first ligation component, XSH is a branched amino acid side chain having a removable thiol auxiliary and is selected from the group consisting of a β-mercapto auxiliary of the formula -CH(R')-SH and a γ-mercapto auxiliary of the formula -CH₂-CH(R')-SH, where R' is selected from the group consisting of ethylene, phenyl, hydroxylphenyl, allylthioalkyl, carbamoyl (-C(O)NH2), carbamoylalkyl, indolyl, hydroxycarbonyl, guanidine alkyl, and imidazolyl,
and J₂ is a residue of a second ligation component.

Reference to the remaining portions of the specification, including the Figures and claims, will realize other features and advantages of the present invention. Further features and advantages of the present invention, as well as the structure and operation of various embodiments of the present invention, are described in detail below with respect to the accompanying Figures and schemes.

### BRIEF DESCRIPTION OF THE DRAWINGS

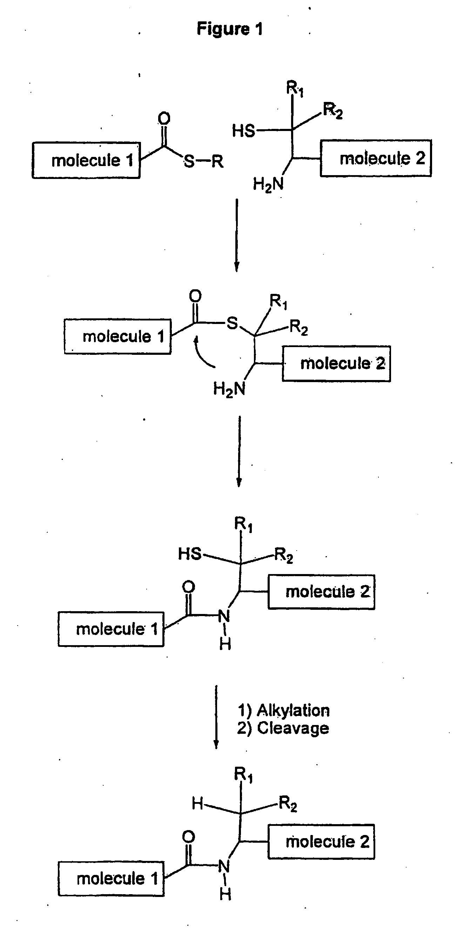

**Fig. 1** illustrates the side chain extended chemical ligation of peptides using a β-branched thiol auxiliary; the same schemes could be employed to effect the ligation of any suitable molecule, including ligation with a γ-branched thiol auxiliary.

**Fig. 2** depicts a synthetic scheme for *anti-*(2*R,3S*)-phenylcysteine derivative.

**Fig. 3** depicts a synthetic scheme for *syn*-(2*R*,3*R*)-phenylcysteine derivative.

**Fig. 4** depicts a coupling scheme for *anti* and *syn* phenylcysteine derivatives and one-pot ligation/alkylation/desulfurization.

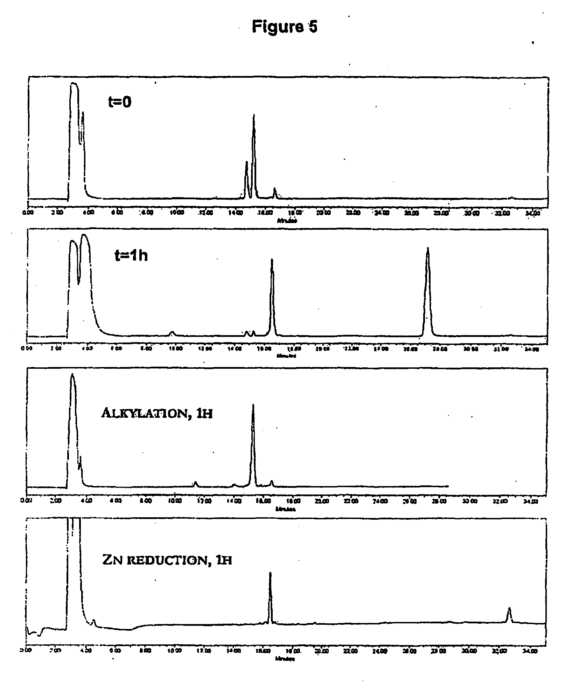

**Fig. 5** illustrates HPLC chromatogram of one-pot ligation/alkylation/desulfurization reaction of the C-terminal peptide LYRAG-thioester with the N-terminal peptide ^{HS}F-YAKYAKL using the *anti*-phenylcysteine (2*R*,*3S*).

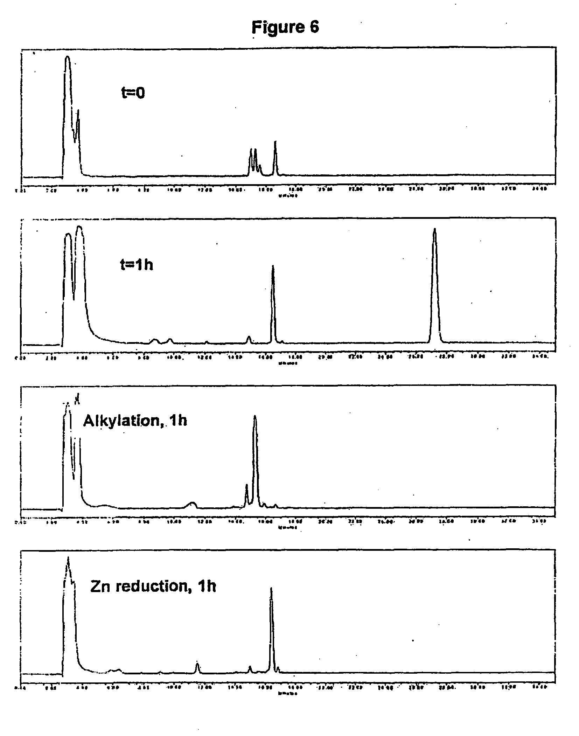

**Fig. 6** illustrates HPLC chromatogram of one-pot ligation/alkylation/desulfurization reaction of the C-terminal peptide LYRAG-thioester with the N-terminal peptide ^{HS}F-YAKYAKL using the *syn*-phenylcysteine (2*R*,*3R*).

### DETAILED DESCRIPTION OF THE INVENTION

The present invention is directed to methods and compositions related to side-chain chemical ligation of a wide range of peptides, polypeptides, other polymers and other molecules. In particular, the present invention provides methods for ligating components to form amide bonds.

**I. Definition**

As used herein, the term "target molecule" refers to a small molecule, a polymer or a surface. Target molecules can be monovalent or multivalent. Such target molecules can be organic molecules (which include molecules of biologic origin as well as organic molecules with inorganic components) having a molecular weight of at least 100 and up to or greater than 10,000. Typically such organic molecules will have a molecular weight of at least 1000, more typically in the range of about 1000-2000. Typically, the target molecule will be a biologically important protein, polypeptides, peptides, amino acids, nucleic acid, liposome, or therapeutic agent. A small molecule means a molecule having the molecular weight less than 1,000, preferably, the small molecule is an organic molecule.

The term "polymer" means a long molecule consisting of structural units and repeating units connected by covalent chemical bonds. The repeating units are small molecules of low to moderate molecular weight, and are linked to each other during a polymerization. The polymers, including oligomers, can be synthetic polymers or naturally occuring polymers. Examples of polymers include, proteins, nucleic acids, liposome and carbohydrates. Typically, a polymer has a molecular weight greater than 1000.

As used herein the term "surface" refers to a two dimensional manifold. Examples of surfaces materials include, materials such as glass, plastic, metal, fiber, and various chromatography materials

As used herein, the term "peptide" refers to two or more amino acids covalently attached through a peptide bond. Peptide is intended to mean both naturally occurring and recombinant forms, as well as other non-naturally occurring forms of the peptide or protein. The non-naturally occurring forms of the peptide can be sufficiently identical to the naturally occurring peptide or protein to allow possession of similar biological or chemical activity. A peptide preferably has from 2 to 50 amino acids. A protein typically can have greater than 50 amino acids. Amino acids which can be used in peptide molecules include those naturally occurring amino acids found in proteins, such as glycine, alanine, valine, cysteine, leucine, isoleucine, serine, threonine, methionine, glutamic acid, aspartic acid, glutamine, asparagine, lysine, arginine, proline, histidine, phenylalanine, tyrosine, and tryptophan. The term amino acid further includes analogs, derivatives and congeners of naturally occurring amino acids, one or more of which can be present in a peptide derivative. For example, amino acids analogs can have lengthened or shortened side chains or variant side chains with appropriate functional groups.

Also included are the D and L stereoisomers of an amino acid when the structure of the amino acid admits of stereoisomeric forms. The term "peptide derivative" further includes compounds which contain molecules which mimic a peptide backbone but are not amino acids (so-called peptidomimetics), such as benzodiazepine molecules (see e.g. James, G. L. et al. Science 1993, 260, 1937-1942).

As used herein, the term "thioester" refers to compounds resulting from the bonding of sulfur with an acyl group, with the general formula R²⁰-C(O)S-R³⁰, where R²⁰ and R³⁰ are each independently a residue of a small molecule, a polymer or a surface.

As used herein, the term "HBTU" refers to O-Benzotriazole-N,N,N',N'-tetramethyl-uronium-hexafluoro-phosphate, a peptide coupling agent.

As used herein the term "TBTU" refers to o-(benzotriazol-1-yl)-N,N,N,N-tetramethyluronium tetrafluoroborate, a peptide coupling agent.

As used herein the term "TCEP" refers to tris(2-carboxyethyl) phosphine, a water soluble reducing agent.

**II. General**

The present invention is directed to methods and compositions related to side chain extended ligation. In one aspect, the side chain extended ligation method of the present invention involves contacting a first component of the formula J₁-C(O)SR with a second component of the formula NH₂-CH(XSH)-J₂ under conditions sufficient to form an amide bond between the first and the second components and to generate a thiol-substituted ligation product of the formula J₁-C(O)-NH-CH(XSH)-J₂. In some embodiments, J₁- is a residue of a first target molecule, polymer or surface; -R can be a residue of a small molecule, a polymer or a surface having functional groups that are compatible with thioesters; Examples of R group include, aryl, benzyl, alkyl, and sulfur protecting group as described in "Protecting Groups in Organic Synthesis", 3rd Edition, T.W. Greene and P.G. M. Wuts, Eds., John Wiley & Sons, Inc., 1999; XSH is an optionally branched amino acid side chain that includes a removable thiol auxiliary and is selected from the group consisting of a β-mercapto auxiliary of the formula -CH(R')-SH and a γ-mercapto auxiliary of the formula -CH₂-CH(R')-SH; R' is selected from the group consisting of ethylene, phenyl, hydroxylphenyl, allylthioalkyl, carbamoyl (-C(O)NH2), carbamoylalkyl, indolyl, hydroxycarbonyl, guanidine alkyl, and imidazolyl and J₂ is a residue of a second target molecule. Minimal lengths for J₁ and/or J₂ could are preferred to be at least 10, 15, 20, 30 or 40 residues length. One of skill in the art will appreciate that functional groups which may interfere with ligation reactions can be suitably protected.

The present invention is also directed to a method that can further include the step of removing the thiol auxiliary to generate a ligation product of the formula J₁-C(O)-NH-CH(R')-J₂. Preferably, J₁-, R', and -J₂ are as defined above.

The compositions of the present invention are directed to a precursor compound, for example a component, which comprises the formula P₁-NH-CH(XSH)-J₂, as well as compositions that comprise a thiol-substituted ligation product having the formula J₁-C(O)-NH-CH(XSH)-J₂. In some preferred embodiments, P₁ is a removable amino protecting group or hydrogen; -CH(XSH), J₁- and -J₂ are as defined above. Suitable amino protecting groups are described in "Protecting Groups in Organic Synthesis", 3rd Edition, T.W. Greene and P.G. M. Wuts, Eds., John Wiley & Sons, Inc., 1999. In another aspect, the present invention is also directed to kits that include as one or more components, one or more compositions of the invention.

The methods and compositions of the present invention expand the scope of amide-forming chemical ligation, and the starting, intermediate and final products of the invention have found a wide range of uses. One advantage is that J₁, J₂ or both J₁ and J₂ in the formulas can be composed of or otherwise joined to join a wide range of chemical moieties, including amino acids, peptides, polypeptides, nucleic acids or other chemical moieties such as dyes, haptens, carbohydrates, lipids, solid support, biocompatible polymers or other polymers and the like. Another advantage is that the amide-forming ligation chemistry of the invention is robust and can be performed in a system near neutral pH and under a range of aqueous conditions. Yet another advantage is the ability of the present invention to conduct "one pot" ligation and thiol auxiliary removal, as well as retention or removal of other thiol protecting groups when they are present under compatible conditions. Still another advantage is the fast ligation reaction rate and high yield of ligation products.

**III. Methods of Ligation**

In one aspect, the method of the present invention involves contacting a first component of the formula J₁-C(O)SR with a second component of the formula NH₂-CH(XSH)-J₂ under conditions sufficient to form an amide bond between the first and second components and to generate a thiol-substituted ligation product of the formula J₁-C(O)-NH-CH(XSH)-J₂. In one embodiment, the method can further include the step of removing the thiol auxiliary to generate a ligation product of the formula J₁-C(O)-NH-CH(XH)-J₂, where XH is an amino acid side chain having a formula selected from the group consisting of - CH₂(R') and -CH₂-CH₂(R'). The method can be illustrated as follows:
(a) J₁-C(O)SR + NH₂-CH(XSH)-J₂
(b) J₁-C(O)-NH-CH(XSH)-J₂
(c) J₁-C(O)-NH-CH(XH)-J₂

In some embodiments, J₁- is a residue of a first target molecule. J₁ can be any chemical moiety compatible with the chemoselective ligation reaction, such as a protected or unprotected amino acid, peptide, polypeptide, other polymer, dye, linker and the like. -C(O)-is a carbonyl group, -S- is a sulfur group, -R is a residue of a small molecule, a polymer or a surface having functional groups that are compatible with thioesters. Examples ofR group include, aryl, benzyl, alkyl, and sulfur protecting group as described in "Protecting Groups in Organic Synthesis", 3rd Edition, T.W. Greene and P.G. M. Wuts, Eds., John Wiley & Sons, Inc., 1999. In certain embodiments, R group can be C₁₋₁₅ functionalized alkyl, straight or branched; C₁₋₁₅ aromatic structures; 20 amino acids or derivatives thereof; and peptides with molecular weight greater than 1000. -XSH is a branched amino acid side chain that includes a removable thiol auxiliary and is selected from the group consisting of a β-mercapto auxiliary of the formula -CH(R')-SH and a γ-mercapto auxiliary of the formula - CH₂-CH(R')-SH, where R' is as defined above. J₂ is a residue of second target molecule. Target molecules J₁ and J₂ can be the same or different, such as a residue of a small molecule, polymer, or surface.

In some embodiments, J₁ and J₂ are each independently a residue of a small molecule, a polymer, or a surface. Minimal lengths for J₁ and/or J₂ are preferred to be at least 10, 15, 20, 30, 40 residues length. Lengths for J₁ and/or J₂ can be 45, 50, 60, 70, 80, 90 or 100 residues length. Examples of preferred J₁ and J₂ groups include a residue of a peptide or polypeptide, which may optionally include one or more protected amino acid side chains. The residue of a peptide or a polypeptide can be composed of amino acids selected from the group consisting of naturally occurring amino acids, analogs, derivatives, congeners, non-naturally occurring amino acids and combinations thereof. Preferred residue of a peptide can comprise amino acids ranging from about 10-20, 20-30, 25-35, 30-40, 35-50, 40-60, 50-80 or 50-100.

Examples of small molecules include, permanent or removable protecting groups, dyes, fluorophores, linkers, detergents, tracers, small molecule drugs and various small organics such as amino acids, nucleotides, nucleosides, sugars, lipids and the like. Polymers include, peptides, polypeptides, nucleic acids, lipids, carbohydrates, fibers and the like. Examples of surfaces include, materials such as glass, plastic, metal, fiber, and various chromatography materials, and preferably are suitable for attachment of a functionalized amino acid.

R can be any group compatible with the thioester group, including, but not limiting to, aryl, benzyl, alkyl groups and sulfur protecting groups. Examples of R include 3-carboxy-4-nitrophenyl thioesters, benzyl thioesters, and mercaptopropionic acid leucine thioesters (See, e.g., Dawson et al., Science 1994, 266, 776-779; Canne et al. Tetrahedron Lett. 1995, 36,1217-1220; Kent, et al., WO 96/34878; Kent, et al., WO 98/28434; Ingenito et al., J. Am. Chem. Soc. 1999, 121, 11369-11374; and Hackeng et al., Proc. Natl. Acad Sci. U.S.A. 1999, 96, 10068-10073). Other examples include dithiothreitol, or alkyl or aryl thioesters, which can be produced by intein-mediated biological techniques, which are also well known (See, e.g., Chong et al., Gene 1997, 192, 277-281; Chong et al., Nucl. Acids Res. 1998, 26, 5109-5115; Evans et al., Protein Science 1998, 7, 2256-2264; and Cotton et al., Chemistry & Biology 1999, 6, 247-256).

In some embodiments, the carboxythioesters can be generated by chemical or biological methods following standard techniques known in the art, such as those described herein, including the Examples. For chemical synthesis, carboxythioester peptides can be synthesized in solution or from thioester-generating resins, which techniques are well known (See, e.g., Dawson et al., supra; Canne et al., supra; Hackeng et al., supra, Hojo H, Aimoto, S. (1991) Bull Chem Soc Jpn 64:111-117). For instance, chemically synthesized thioester peptides can be made from the corresponding peptide-α-thioacids, which in turn, can be synthesized on a thioester-resin or in solution, although the resin approach is preferred. The peptide-α-thioacids can be converted to the corresponding 3-carboxy-4-nitrophenyl thioesters, to the corresponding benzyl ester, or to any of a variety of alkyl thioesters. All of these thioesters provide satisfactory leaving groups for the ligation reactions, with the 3-carboxy-4-nitrophenyl thioesters demonstrating a somewhat faster reaction rate than the corresponding benzyl thioesters, which in turn may be more reactive than the alkyl thioesters. As another example, a trityl-associated mercaptoproprionic acid leucine thioester-generating resin can be utilized for constructing C-terminal thioesters (Hackeng et al., supra). C-terminal thioester synthesis also can be accomplished using a 3-carboxypropanesulfonamide safety-catch linker by activation with diazomethane or iodoacetonitrile followed by displacement with a suitable thiol (Ingenito et al., supra; Shin et al., (1999) J. Am. Chem. Soc., 121, 11684-11689). Thioesters compounds may also be constructed for syntheses that require one or more steps involving nucleophilic conditions, such as with Fmoc-based peptide synthesis (WO 02/18417; WO 03/106615), or for thioester compounds with acidic groups in reactive proximity to the thioester moiety (WO 04/007661).

Peptide or polypeptide C-terminal carboxythioesters also can be made using biological processes. For instance, intein expression systems, with or without labels such as affinity tags can be utilized to exploit the inducible self-cleavage activity of an "intein" protein-splicing element in the presence of a suitable thiol to generate a C-terminal thioester peptide or polypeptide segment. In particular, the intein undergoes specific self-cleavage in the presence of thiols such as DTT, β-mercaptoethanol, β-mercaptoethanesulfonic acid, or cysteine, which generates a peptide segment bearing a C-terminal thioester. See, e.g., Chong et al., (1997) supra; Chong et al., (1998) supra; Evans et al., supra; and Cotton et al., supra; WO 03/106 615; WO 02/098902.

Ligation of the thiol-bearing component of the invention with the carboxythioester-bearing component generates a ligation product having an amide bond at the ligation site and a removable thiol auxiliary. The ligation conditions of the reaction are chosen to maintain the selective reactivity of the thioester with the thiol moiety. In a preferred embodiment, the ligation reaction is carried out in a buffer solution having pH 6-8, with the preferred pH range being 6.5-7.5. The buffer solution may be aqueous, organic or a mixture thereof. The ligation reaction also can include one or more catalysts and/or one or more reducing agents, lipids, detergents, other denaturants or solubilizing reagents and the like. Examples of preferred catalysts are thiol, imidazole and phosphine containing moieties, such as thiophenol, histidine, benzylmercaptan, TCEP and alkyl phosphines. Examples of denaturing and/or solubilizing agents include guanidinium, urea in water or organic solvents such as TFE, HFIP, DMF, NMP, acetonitrile admixed with water, or with guanidinium and urea in water. The temperature also may be utilized to regulate the rate of the ligation reaction; which is usually between 5°C and 55°C, with the preferred temperature being between 15°C and 40°C. As an example, the ligation reactions proceed well in a reaction system having 2% thiophenol in 6M guanidinium at a pH between 6.8 and 7.8.

Removal of the thiol auxiliary group is preferably performed after completion of the ligation reaction. In a preferred method, the thiol is alkylated followed by hydrolysis to generate a ligation product from which the thiol auxiliary is removed, such as depicted in Scheme I (as illustrated for the β-branched auxiliary thiol derivatives) in which alkylation is preferably carried out using a halide-functionalized alkyl group, such as bromoacetamide or iodoacetamide. Following alkylation (or concurrent with), the modified thiol auxiliary can be removed by hydrolysis, such as with zinc-acetic acid to facilitate cleavage of the thiol bond as illustrated in Scheme II, yielding a stabilized, unsubstituted side chain for the residue at the ligation site.

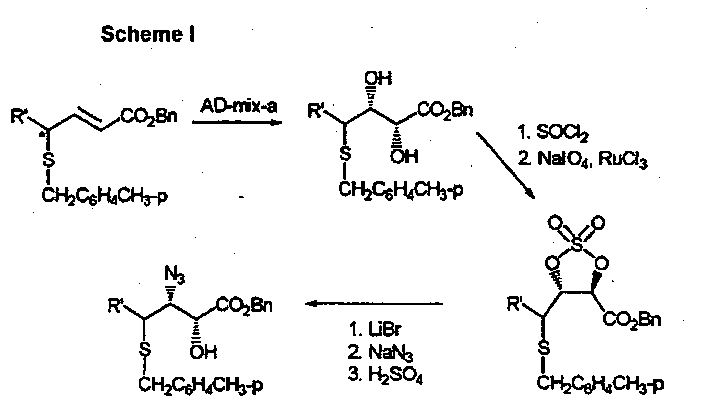

As can be appreciated, one or more catalysts and/or excipients may also be utilized in the cleavage system, such as one or more scavengers, detergents, solvents, metals and the like. In general, selection of specific scavengers depends upon the amino acids present. For instance, the presence of scavengers can be used to suppress the damaging effect that the carbonium ions, produced during cleavage, can have on certain amino acids (e.g., Met, Cys, Trp, and Tyr). Other additives like detergents, polymers, salts, organic solvents and the like also may be employed to improve cleavage by modulating solubility. Catalysts or other chemicals that modulate the redox system also can be advantageous.

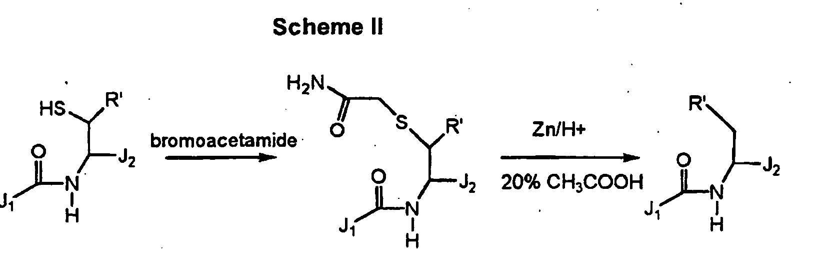

It also will be readily apparent that a variety of other physical-chemical conditions such as buffer systems, pH and temperature can be routinely adjusted to optimize a given cleavage system, such as peptide-compatible cleavage conditions. By "peptide-compatible cleavage conditions" is intended physical-chemical conditions compatible with peptides and suitable for cleavage of the thiol moiety from the ligation product. Peptide-compatible cleavage conditions in general are selected depending thiol moiety employed, which can be readily deduced through routine and well known approaches (See, e.g., "Protecting Groups in Organic Synthesis", 3rd Edition, T.W. Greene and P.G.M. Wuts, Eds., John Wiley & Sons, Inc., 1999; NovaBiochem Catalog 2000; "Synthetic Peptides, A User's Guide," G.A. Grant, Ed., W.H. Freeman & Company, New York, NY,1992; "Advanced Chemtech Handbook of Combinatorial & Solid Phase Organic Chemistry," W.D.. Bennet, J.W. Christensen, L.K. Hamaker, M.L. Peterson, M.R.Rhodes, and H.H. Saneii, Eds., Advanced Chemtech, 1998; "Principles of Peptide Synthesis, 2nd ed.," M. Bodanszky, Ed., Springer-Verlag, 1993; "The Practice of Peptide Synthesis, 2nd ed.," M. Bodanszky and A. Bodanszky, Eds., Springer-Verlag, 1994; and "Protecting Groups," P.J. Kocienski, Ed., Georg Thieme Verlag, Stuttgart, Germany, 1994).

The ligation method of the present invention can be used for the preparation of various therapeutically important proteins including, but not limiting to, monoclonal and polyclonal antibodies; antiviral proteins; hormones such as insulin; cytokines such as tumor necrosis factor, including colony stimulating factors such as M-CSF, GM-CSF and G-CSF; stem-cell growth factor; lymphokines; IL-2 and IL-3; growth factors, including, PDGF, EGF; peptide hormones, including hGH and erythropoietin; blood clotting factors, including Factor VIII; immunogens; enzymes and enzyme inhibitors; ligands; vaccine antigens; plasma proteins such as fibrinogen, immunoglobulins or fragments thereof; enzymes such as tissue plasminogen activator; and the like.

**IV. Compositions**

In one embodiment, the compositions of the present invention include a β-branched or γ-branched amino acid residue bearing a side chain substituted with a removable thiol auxiliary, as well as precursors, intermediates, and ligation products comprising such a residue. A preferred amino acid composition comprises a β-branched thiol auxiliary (i.e., 2-mercapto amino) of Formula I:

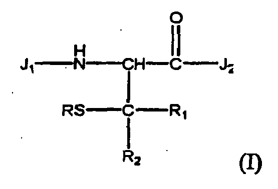

In some preferred embodiments, J₁ and J₂ are each independently a residue of a small molecule, a polymer, or a surface; R is hydrogen or thiol protecting group; R₁ and R₂ are each independently hydrogen or an organic group, with the proviso that one of R₁ and R₂ is selected from ethylene, phenyl, hydroxylphenyl, allylthioalkyl, carbamoyl (-C(O)NH2), carbamoylalkyl, indolyl, hydroxycarbonyl, guanidine alkyl, and imidazolyl. One of skill in the art will appreciate that functional groups which may interfere with ligation reactions can be suitably protected. Examples of small molecules are permanent or removable protecting groups, dyes, fluorophores, linkers, detergents, tracers, small molecule drugs and various small organics such as amino acids, nucleotides, nucleosides, sugars, lipids and the like. Polymers include peptides, polypeptides, nucleic acids, lipids, carbohydrates, fibers and the like. Examples of surfaces include materials such as glass, plastic, metal, fiber, and various chromatography materials, and preferably are suitable for attachment of a functionalized amino acid. Examples of preferred J₁ and J₂ groups include a residue of a peptide or polypeptide, which may optionally include one or more protected amino acid side chains.

In another embodiment, the invention is directed to an amino acid composition comprising a γ-branched thiol auxiliary (i.e., 3-mercapto amino), and comprises Formula II: 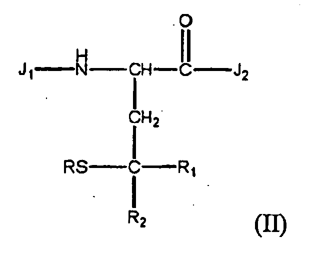 where J₁, J₂, R, R₁ and R₂ are as defined above.

Synthesis of compounds according to Formula I and Formula II of the invention can be carried out as described herein, and in accordance with standard organic chemistry techniques known in the art. See, e.g., "Advanced Organic Chemistry, Reactions, Mechanisms, and Structure," 4th Edition, J. March (Ed.), John Wiley & Sons, New York, NY, 1992; "Comprehensive Organic Transformations, A Guide to Functional Group Preparations," R. Larock (Ed.), VCH Publishers, New York, NY, 1989. They may be synthesized in solution, by polymer-supported synthesis, or a combination thereof. The reagents utilized for synthesis can be obtained from any number of commercial sources. Also, it will be well understood that the starting components and various intermediates, such as the individual amino acid derivatives can be stored for later use, provided in kits and the like.

Preferred purity is at least 75, 80, 85, 90, 95 and 100% purity of the desired ligated compound in the ligation compositions prior to subsequent purification or isolation of the desired compound.

Synthesis may employ protecting group strategies. The preferred protecting groups (PG) utilized in the various synthesis strategies in general are compatible with Solid Phase Peptide Synthesis ("SPPS"). In some instances, it also is necessary to utilize orthogonal protecting groups that are removable under different conditions. Many such protecting groups are known and suitable for this purpose (See, e.g., "Protecting Groups in Organic Synthesis", 3rd Edition, T.W. Greene and P.G.M. Wuts, Eds., John Wiley & Sons, Inc., 1999; NovaBiochem Catalog 2000; "Synthetic Peptides, A User's Guide," G.A. Grant, Ed., W.H. Freeman & Company, New York, NY,1992; "Advanced Chemtech Handbook of Combinatorial & Solid Phase Organic Chemistry," W.D.. Bennet, J.W. Christensen, L.K. Hamaker, M.L. Peterson, M.R.Rhodes, and H.H. Saneii, Eds., Advanced Chemtech, 1998; "Principles of Peptide Synthesis, 2nd ed.," M. Bodanszky, Ed., Springer-Verlag, 1993; "The Practice of Peptide Synthesis, 2nd ed.," M. Bodanszky and A. Bodanszky, Eds., Springer-Verlag, 1994; and "Protecting Groups," P.J. Kocienski, Ed., Georg Thieme Verlag, Stuttgart, Germany, 1994). Examples include benzyloxycarbonyl (Z), Boc, Bpoc, Trt, Nps, FmocCI-Z, Br-Z; NSC; MSC, Dde, etc. For sulfur moieties, examples of suitable protecting groups include, but are not limited to, benzyl, 4-methylbenzyl, 4-methoxybenzyl, trityl, Acm, TACAM, xanthyl, disulfide derivatives, picolyl, and phenacyl.

Preferred compounds according to Formula I include protected amino acid derivatives synthesized according to Scheme III that may be conveniently prepared as a protected derivative ready for peptide or other syntheses of interest, as illustrated for the *tert-*butyloxycarbonyl (Boc) and p-methylbenzyl (ρ-CH₃-C₆H₄CH₂-) protected derivatives, where R' is a protected or unprotected amino acid side chain of interest. For instance, where compatibility with Boc-based peptide synthesis is desired, an amino acid side chain of interest such as a phenylalanine (Phe, or F) side chain can be unprotected through synthesis, whereas a tyrosine (Tyr, or Y) side chain could bear *O*-2-bromobenzyloxycarbonyl (2BrZ) group. Referring to Scheme III, synthesis may employ regioisomers (as shown) or a single isomer. In accordance with Scheme III in general, triphenylphosphine is added to the desired azido alcohol(s) in an appropriate solvent (e.g., CH₃CN), followed by reflux, removal of solvent and purification of the aziridine. The aziridine is modified with the auxiliary thiol by addition of 4-methylbenzylthiol in an appropriate solvent and in the presence of a mild Lewis acid catalyst (e.g. boron trifluoride etherate). The desired p-methylbenzene sulfonyl protected intermediate can then be purified by standard methods from the organic layer, followed addition of the N-terminal protecting group (e.g., Boc), and deprotection of the carboxyl group by standard methods.

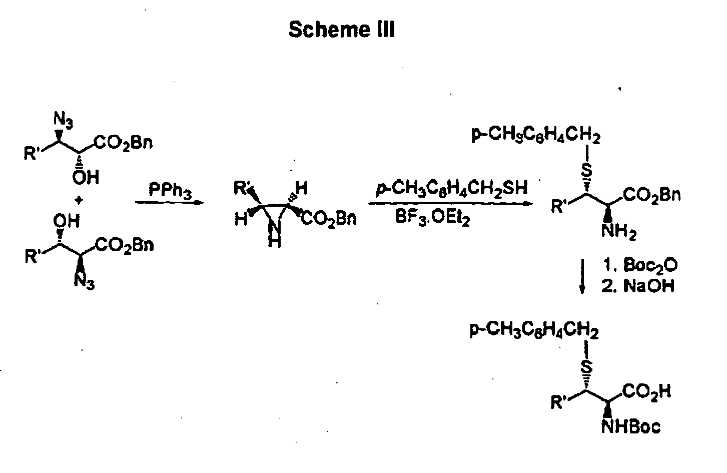

Preferred compounds according to Formula II include protected amino acid derivatives synthesized according to Scheme I that may be conveniently prepared as a protected derivative ready for peptide or other syntheses of interest. Referring to Scheme I, the (E)-phenyl 4-(ρ-tolylthio)-4-R'-2-enoate is prepared with the desired amino acid side chain R' (e.g., benzyl / Phe side chain), and added to an aqueous solution of AD-mix-α and methanesulfonamide to form the cyclic sulfate. The cyclic sulfate is then converted to the azide and further prepared as in Scheme III..

The present invention also provides protected forms of the side chain auxiliary thiol compositions of the invention. These compounds are especially useful for automated peptide synthesis and orthogonal and convergent ligation strategies. These compositions comprise a fully protected, as well as partially protected compounds. Many such protecting groups are known and suitable for this purpose (See, e.g., "Protecting Groups in Organic Synthesis", 3rd Edition, T.W. Greene and P.G.M. Wuts, Eds., John Wiley & Sons, Inc., 1999; NovaBiochem Catalog 2000; "Synthetic Peptides, A User's Guide," G.A. Grant, Ed., W.H. Freeman & Company, New York, NY,1992; "Advanced Chemtech Handbook of Combinatorial & Solid Phase Organic Chemistry," W.D. Bennet, J.W. Christensen, L.K. Hamaker, M.L. Peterson, M.R.Rhodes, and H.H. Saneii, Eds., Advanced Chemtech, 1998; "Principles of Peptide Synthesis, 2nd ed.," M. Bodanszky, Ed., Springer-Verlag, 1993; "The Practice of Peptide Synthesis, 2nd ed.," M. Bodanszky and A. Bodanszky, Eds., Springer-Verlag, 1994; and "Protecting Groups," P.J. Kocienski, Ed., Georg Thieme Verlag, Stuttgart, Germany, 1994).

The side chain auxiliary thiol compositions of the invention can be employed in combination with various ligation methods, for example, such as native chemical ligation (Dawson, et al., Science (1994) 266:776-779; Kent, et al., WO 96/34878), extended general chemical ligation (Kent, et al., WO 98/28434), oxime-forming chemical ligation (Rose, et al., J. Amer. Chem. Soc. (1994) 116:30-33), thioester forming ligation (Schnölzer, et al., Science (1992) 256:221-225), thioether forming ligation (Englebretsen, et al., Tet. Letts. (1995) 36(48):8871-8874), hydrazone forming ligation (Gaertner, et al., Bioconj. Chem. (1994) 5(4):333-338), and thiazolidine forming ligation and oxazolidine forming ligation (Zhang, et al., Proc. Natl. Acad. Sci. (1998) 95(16):9184-9189; Tam, et al., WO 95/00846) or by other methods (Yan, L.Z. and Dawson, P.E., "Synthesis of Peptides and Proteins without Cysteine Residues by Native Chemical Ligation Combined with Desulfurization," J. Am. Chem. Soc. 2001, 123, 526-533, Gieselnan et al., Org. Lett. 2001 3(9):1331-1334; Saxon, E. et al., "Traceless" Staudinger Ligation for the Chemoselective Synthesis of Amide Bonds. Org. Lett. 2000, 2, 2141-2143).

The present invention also contemplates compositions of grafting polymers onto the ligation products. It is well recognized that the properties of peptides or proteins can be enhanced by grafting organic chain-like molecules onto them. These molecules are often polyethylene glycol-based ("PEG"-based, i.e. based on the repeating unit -CH₂CH₂O-). See for example, Tsutsumi, et al, Jpn. J. Cancer Res. 1994, 85, 9-12. PEG-based chains are amphiphilic, non-immunogenic and not susceptible to cleavage by proteolytic enzymes. Preparations of materials that have been modified by PEG or PEG-based chains, have reduced immunogenicity and antigenicity. See for example, Abuchowski, et al, Journal of Biological Chemistry 1977, 252, 3578-3581. PEG also serves to increase the molecular size of the material to which it is attached, thereby increasing its biological half-life. These beneficial properties of the PEG-modified materials make them very useful in a variety of therapeutic applications. The following patent applications report PEG-based modifications of various biologically important proteins: U.S. Pat. Nos. 4,179,337; 4,609,546; 4,261,973; 4,055,635; 3,960,830; 4,415,665; 4,412,989; 4,002,531; 4,414,147; 3,788,948; 4,732,863; and 4,745,180; EP No. 152,847; EP98110 published Jan. 11, 1984; and JP5792435. Polyamide chains also are useful as organic chain-like molecules to enhance properties. In addition, acute toxicity screening in rodents suggests that polyamides are neither toxic nor immunogenic (Hai, et al., Bioconj. Chem. 1998, 9, 645-654).

The grafting of PEG chains or PEG-based chains onto proteins is known. See for example, Zalipsky, U.S. Pat. No. 5,122,614, which describes PEG that is converted into its N-succinimide carbonate derivative. Also known are PEG chains modified with reactive groups to facilitate grafting onto proteins. See for example, Harris, U.S. Pat. No. 5,739,208, which describes a PEG derivative that is activated with a sulfone moiety for selective attachment to thiol moieties on molecules and surfaces and Harris, et al., U.S. Pat. No. 5,672,662, which discloses active esters of PEG acids that have a single propionic or butanoic acid moiety. This area is extensively reviewed in Zalipsky, Bioconjugate Chemistry 1995, 6, 150-165 (1995). Besides use of PEG, Wright, EP 0 605 963 A2 describes linking reagents that contain water soluble polymers that form a hydrazone linkage with an aldehyde group on a protein.

The methods and compositions of the invention have many uses. The methods and compositions of the invention are particularly useful for ligating peptides, polypeptides and other polymers.

### EXAMPLES

The following preparations and examples are given to enable those skilled in the art to more clearly understand and to practice the present invention. They should be considered merely as being illustrative and representative thereof.

### Example 1: General Materials and Methods

Peptides were synthesized in stepwise fashion on a modified ABI 433A peptide synthesizer by SPPS using in situ neutralization/HBTU activation protocols for Boc-chemistry on PAM resin or thioester-generating resin following standard protocols (Hackeng et al., supra; Schnolzer et al., Int. J. Pept. Prot. Res., 1992, 40, 180-193; and Kent, S.B.H. Ann. Rev. Biochem. 1988, 57, 957-984). After chain assembly the peptides were deprotected and simultaneously cleaved by treatment with anhydrous hydrogen fluoride (HF) with 5% p-cresol and lyophilized and purified by preparative HPLC.

### Example 2: Design of anti- and syn-phenyl cysteines

As an initial test residue for the side chain extended chemical ligation method of the invention, phenylalanine residue(s) ready to be incorporated onto a peptide fragment were constructed. With the presence of a mercaptan on the benzylic position, there were two possible geometric isomers that could employed for ligation:

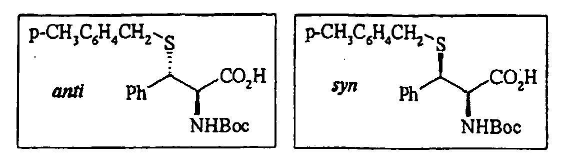

The two isomers, by having the two reacting moieties (mercaptan and the amine) on the same side (*syn* phenylcysteine) or on opposite sides (*anti* phenylcysteine) might have different properties either in ligation or during the desulphurisation/cleavage step or in both. Thus, the synthesis of the two isomers is necessary to find the best isomer (both ligation and cleavage steps) to be used in our strategy.

### Example 3: Preparation of (2R,3S)-2,3-dihydroxy-3-phenylpropionic acid benzyl ester

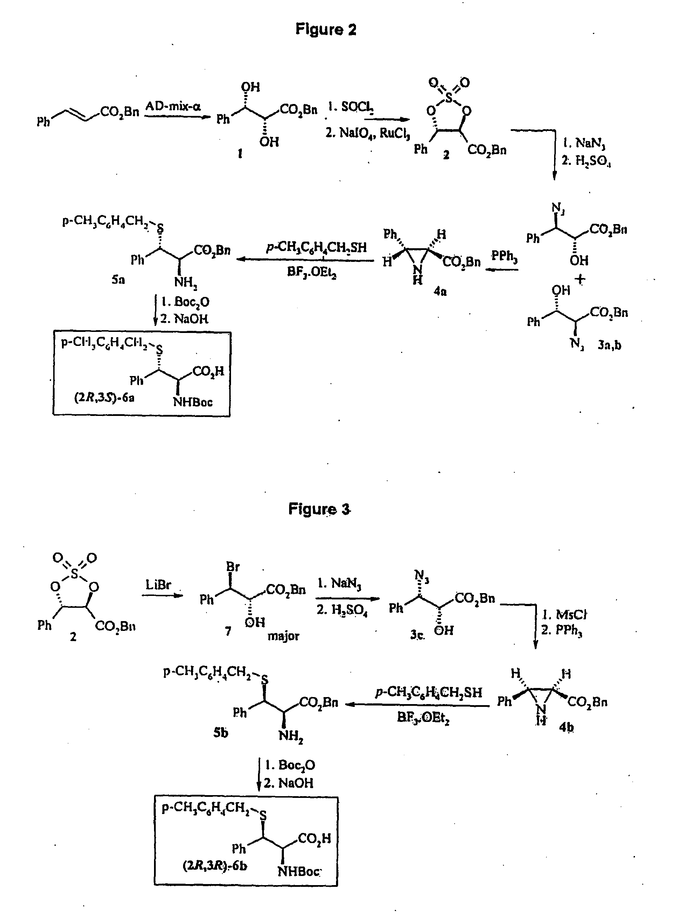

Referring to Figure 2, to a stirred solution of AD-mix-α (9.8 g) and methanesulfonamide (666 mg, 7 mmol) in t-BuOH (ml) and water (ml) at room temperature was added benzyl cinnamate (1.67 g, 7 mmol). The resulting mixture was stirred at room temperature for 24 h. Sodium sulfite (10.5 g) was added, and the mixture was stirred for 30 mn, diluted with ethyl acetate, washed with water. Organic layer was dried over MgSO₄, filtered and concentrated. Flash chromatography (ethyl acetate/pentane 1/2) on silica gel afforded compound 1 (1.55 g, 81 %) as a white solid.

### Example 4: Preparation of cyclic sulfate

Referring to Figures 2 and 3, to an ice-cold stirred solution of diol 1 (680 mg, 2.5 mmol) and triethylamine (695 µl, 5 mmol) in dichloromethane (5 ml) was added thionyl chloride (275 µl, 3.75 mmol) dropwise over a period of 10 mn. Mixture was stirred 10-15 mn, diluted with cold ether and washed with cold water. Aqueous layer was extracted with ether. The combined organic layers were dried over MgSO₄, filtered and concentrated. The crude cyclic sulfite was dissolved in a mixture of water (14 ml), CH₃CN (7 ml) and CCl₄ (7 ml). Sodium periodate (802 mg, 3.75 mmol) and RuCl₃ hydrate (catalytic amount) were added and the obtained mixture was vigorously stirred for 1 h at room temperature, diluted with ether, and the organic layer was filtered through a pad of celite. The organic layer was washed with water, saturated NaHCO₃ solution and brine, dried over MgSO₄, filtered and concentrated. Flash chromatography (ethyl acetate/pentane 1/4) on silica gel afforded compound 2 (568 mg, 63%) as a colorless oil.

### Example 5: Preparation of a mixture of regioisomers (2S,3S)-2-azido-3-hydroxy-3-phenylpropionic acid benzyl ester and (2R,3R)-3-azido-2-hydroxy-3-phenylpropionic acid benzyl ester.

Referring to Figure 2, to a stirred solution of cyclic sulfate **2** (568 mg, 1.7 mmol) in acetone (10 ml) and water (1 ml) was added NaN₃ (138 mg, 2.13 mmol) as a solid. The reaction mixture was stirred for 5 min at 0 °C then at room temperature for 2 h, and concentrated under reduced pressure. Ether (7 ml) and water (2 ml) were added, and the solution was chilled to 0 °C, followed by dropwise addition of H₂SO₄ 20% aqueous solution (7 ml). The resulting mixture was stirred at 0 °C for 1 h, then allowed to slowly warm to room temperature and stirred at room temperature for 3 h. The organic layer was collected, washed with saturated NaHCO₃ solution and water, dried over MgSO_{4,} filtered and concentrated. Flash chromatography (ethyl acetate/pentane 1/4) on silica gel afforded mixture of regioisomers **3a,b** (384 mg, 76%) as a white solid.

### Example 6: Preparation of (2S,3R)-3-phenylaziridine-2-carboxylic acid benzyl ester

Referring to Figure 2, to a stirred solution of azido alcohols **3a,b** (384 mg, 1.29 mmol) in CH₃CN (6 ml) was added at room temperature triphenylphosphine (351 mg, 1.34 mmol) as a solid. The mixture was stirred at room temperature for 1 h then refluxed for 6 h. After removal of solvent, pure aziridine **4** (250 mg, 77 %) was isolated as a white solid by flash chromatography (ethyl acetate/pentane 1/4) on silica gel.

### Example 7: Preparation of (2R,3S)-2-(tert-Butyloxycarbonylamino)-3-(4-methylbenzylsulfanyl)-3-phenylpropionic Acid

Referring to Figure 2, to a stirred solution of aziridine **4** (125 mg, 0.5 mmol) and 4-methylbenzylthiol (135 µl, 1 mmol) in dichloromethane (5 ml) at 0 °C was added boron trifluoride etherate (95 µl, 0.75 mmol) dropwise. Mixture was stirred at 0 °C for 6-8 h and then allowed to slowly warm to room temperature overnight. The reaction was then quenched by addition of saturated NaHCO₃ and the organic layer was washed with brine, water, dried over MgSO₄, filtered and concentrated. Flash chromatography (ethyl acetate/pentane 1/2) on silica gel afforded compound **5a** (150 mg, 77%) as a colorless oil. This product was dissolved in THF (3 ml). The solution was treated with NEt₃ (54 µl, 0.39 mmol), and Boc₂O (84 mg, 0.38 mmol) was added. The reaction mixture was then stirred overnight at room temperature, concentrated. The crude residue is dissolved in dioxane (2.2 ml) and a 4 M NaOH solution (475 µl, 1.90 mmol) was added. The resulting mixture was stirred for 17 h at room temperature. Water was added and the aqueous layer extracted with ethyl acetate, then acidified with a 1M HCl solution and extracted with ethyl acetate. The combined organic layers were dried over MgSO₄, filtered and concentrated. Flash chromatography (ethyl acetate/pentane 1/1) on silica gel afforded compound **(2*R*,*3S*)-6a** (85 mg, 62%) as a white solid.

### Example 8: Preparation of (2S,3R)-3-bromo-2-hydroxy-3-phenylpropionic acid benzyl ester

Referring to Figures 2 and 3, to a stirred solution of cyclic sulfate 2 (2.1 g, 6.29 mmol) in THF (88 ml) was added at room temperature solid LiBr (2.07 g, 23.89 mmol). The resulting mixture was stirred overnight at room temperature, then concentrated. Ether (30 ml) and water (5 ml) were added, and the solution was chilled to 0 °C, followed by dropwise addition of H₂SO₄ 20% aqueous solution (40 ml). The mixture was stirred at 0 °C for 2-3 h then at room temperature for 6h. The organic layer was collected, washed with saturated NaHCO₃ solution and water, dried over MgSO₄, filtered and concentrated. Flash chromatography (ethyl acetate/pentane 1/4) on silica gel afforded compound 7 (1.83 g, 87%) as a white solid.

### Example 9: Preparation of (2R,3S)-3-azido-2-hydroxy-3-phenylpropionic acid benzyl ester

Referring to Figures 2 and 3, a stirred solution of bromide **7** (1.73 g, 5.16 mmol) in DMSO (20 ml) was added solid NaN₃ (671 mg, 10.33 mmol). The mixture was stirred at room temperature for 24 h, then diluted with pentane/dichloromethane 1/1 mixture and washed with water. The organic layer was dried over MgSO₄, filtered and concentrated. Flash chromatography (ethyl acetate/pentane 1/4) on silica gel afforded compound **3b** (1.13 g, 74%) as a white solid.

### Example 10: Preparation of (2S,3S)-3-phenylaziridine-2-carboxylic acid benzyl ester4b

Referring to Figures 2 and 3, to a stirred solution of azido alcohol **3b** (400 mg, 1.35 mmol) in dichloromethane (15 ml) were added at room temperature NEt₃(375 µl, 2.70 mmol) followed by methanesulfonyl chloride (160 µl, 2.03 mmol). The resulting mixture was stirred at room temperature for 2 h, diluted with ethyl acetate and washed with water. The aqueous layer was extracted with ethyl acetate and the combined organic layers were dried over MgSO₄, filtered and concentrated. The crude residue was dissolved in THF/water 10/1 mixture (27 ml). To this solution were added at room temperature DIEA (460 µl, 2.70 mmol) followed by triphenylphosphine (708 mg, 2.70 mmol). The resulting mixture was stirred for 3.5 h at 35°C, cooled to room temperature. Water was added and the aqueous layer was extracted with ethyl acetate. Combined organic layers were dried over MgSO_{4,} filtered and concentrated. Flash chromatography (ethyl acetate/pentane 1/4) on silica gel afforded compound **4** (170 mg, 50%) as a white solid.

### Example 11: Preparation of (2R,3R)-2-(tert-Butyloxycarbonylamino)-3-(4-methylbenzylsulfanyl)-3-phenylpropionic acid

Referring to Figures 2 and 3, the same procedure described in the preparation of **(*2R*,*3S*)-6a** but starting with aziridine **(*2R,3R*)-4b** (170 mg, 0.67 mmol) afforded compound **(*2R,3R*)-6b** (18 mg, 7% for the 3 steps) as a white solid.

### Example 12: General procedure for the coupling of anti-6a and syn-6b derivatives to carboxyester resin and subsequent HF cleavage.

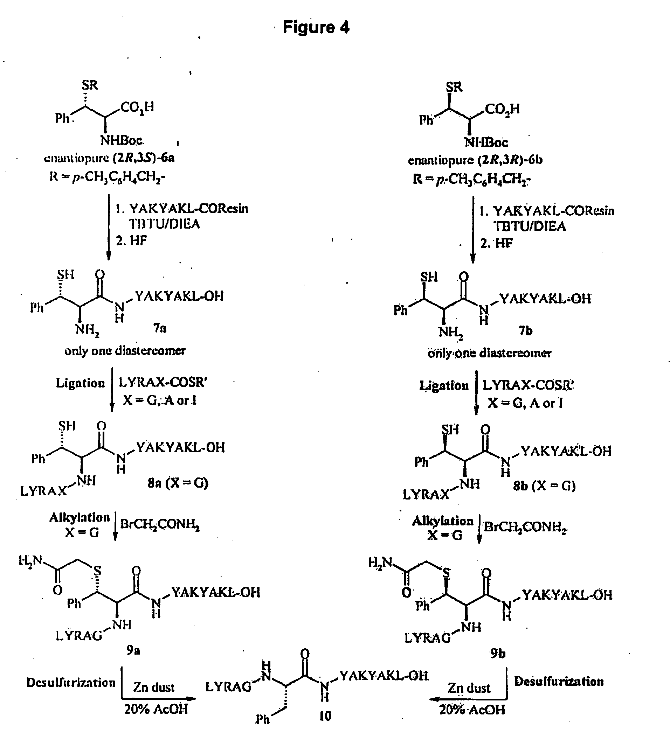

Referring to Figure 4, **(*2R*,*3S*)-6a** or **(*2R,3R*)-6b** (0.11 mmol) were coupled for 1.5 h to peptide resin (0.10 mmol) in DMF in the presence of TBTU (0.11 mmol) and DIEA (0.20 mmol). The coupling completion was monitored by ninhydrin test. The peptides were then deprotected and cleaved from the resin by treatment with anhydrous HF for 1 h at 0°C with 500 µl p-cresol as scavenger. After cleavage, peptides were precipitated with ether, dissolved in acetonitrile/water 50/50 mixture, and lyophilized. Peptides diastereomers **7a** or **7b** obtained respectively from coupling with **(*2R,3S*)-6a** and **(*2R*,*3R*)-6b** were purified by reversed phase HPLC, affording white powders characterized by ESI mass spectrometry. For peptide **7a**: ESI-MS calculated for C51H75N10O11S (M+H) 1035.53, found 1035.55. For peptide **7b**: ESI-MS calculated for C51H75N10O11S (M+H) 1035.53, found 1035.54.

### Example 13: General procedure for one-pot ligation/alkylation/desulfurization reactions

Referring to Figure 4, peptide **7a** (or **7b**) (3 mM final concentration) was dissolved in a phosphate buffer* with peptide thioester LYRAG-COSR (1.25 eq.) and the resulting solution was stirred at 37°C in the presence of thiophenol (0.3 % V/V). Ligation completion was monitored by HPLC analysis: aliquots of this solution were treated with BME (20 % V/V) for 20 minutes and then with TCEP for 5 minutes before injection. (where *buffer contains NaH₂PO₄ 0.2 M, guanidinium 6M, 20 mM Met, pH 7.6).

After completion of the ligation reaction, some TCEP was added to the reaction mixture, to have a pH=3-4. After 5 mn the solution was basified to pH 8, and iodoacetamide or bromoacetamide was added. The resulting mixture was stirred at room temperature (alkylation reaction was monitored by HPLC analysis), then extracted with diethyl ether to remove thiophenol derivatives. Alkylation gave generally quantitative yield on ligation crude. Acetic acid (20% VN) was then added, followed by zinc dust (µspatula) for the desulfurization reaction (monitored by HPLC analysis), which gave ∼98% yield of native product after 1 hr from the "one pot" reaction. Electrospray mass spec analysis was as follows: for peptide **8** - (not isolated intermediate) ESI-MS calcd for C₇₇H₁₁₅N₁₈O₁₇S (M+H) 1595.84, found 1595.90; for peptide - (not isolated intermediate), ESI-MS calcd for C₇₉H₁₁₈N₁₉O₁₈S (M+H) 1652.86, found 1652.85; and for peptide **10** - ESI-MS calcd for C₇₇H₁₁₅N₁₈O₁₇ (M+H) 1563.87, found 1563.82.

As noted, the above reactions were all carried out under conditions compatible with the ligation conditions: 6M Guanidinium, pH 7.6, 37°C, and thus were "One Pot" meaning that no intermediate purification is necessary to obtain the final native product. Importantly, the final product in the "One Pot" reaction was confirmed to be the native peptide (L-residue at the Phe ligation site, no racemization) by comparison with two standard peptides made by solid phase peptide synthesis (SPPS) of sequence LYRAGFYAKYAKL, where the F residue in one control peptide was the natural L-Phe and in the other control peptide the unnatural D-Phe. The ligation product as analyzed by HPCL was found to be identical to the native standard and does not contains traces of the unnatural derivative (data not shown).

### Example 14: Ligation reactions with model peptide thioesters LYRAA-COSR or LYRAI-COSR.

Referring to Figure 4, peptide **7a** (or **7b** or model peptide CYAKYAKL) was dissolved in a phosphate buffer* (3 mM final concentration) with respectively peptide thioester LYRAA-COSR or LYRAI-COSR (1.25 eq.) and the resulting solution was stirred at 37°C in the presence of thiophenol (0.3 % V/V). Ligation completion was monitored by HPLC analysis: aliquots of this solution were treated with BME (20 % VN) for 20 minutes and then with TCEP for 5 minutes before injection (where *buffer contains NaH₂PO₄ 0.2 M, guanidinium 6M, 20 mM Met, pH 7.6).

Ligation tests with *anti* or *syn* phenylcycsteine were compared to Native Chemical Ligation (which uses cysteine) are reported below in Table I and Table II.

**Table I: Ligation Using anti Phenylcysteine**

| Peptide | X (LYRAX-COSR) | Yield after 1 hour (%) | Completion Time (96%)(h) |
|---|---|---|---|
| ^{HS}FYAKYAK | G | 96 | 1 |
| ^{HS}FYAKYAK | A | 90 | 2.5 |
| ^{HS}FYAKYAK | I | 20 | - |
| CYAKYAK | A | 96 | 1 |
| CYAKYAK | I | 40 | - |

**Table II: Ligation Using syn Phenylcysteine**

| Peptide | X (LYRAX-COSR) | Yield after 1 hour (%) | Completion Time (96%)(h) |
|---|---|---|---|
| ^{HS}FYAKYAK | G | 96 | 1 |
| ^{HS}FYAKYAK | A | 96 | 1 |
| ^{HS}FYAKYAK | I | 50 | - |
| CYAKYAK | A | 96 | 1 |
| CYAKYAK | I | 40 | - |

## Claims

1. A method of ligating components through an amide bond, said method comprising:
contacting a component of the formula J₁-C(O)SR with a component of the formula NH₂-CH(XSH)-J₂ under conditions sufficient to form a thiol-substituted ligation product having the formula J₁-C(O)-NH-CH(XSH)-J₂,
wherein
J₁ is a residue of a first target molecule, polymer or surface;
R is a group compatible with thioesters;
XSH is a branched amino acid side chain having a removable thiol auxiliary and selected from the group consisting of a β-mercapto auxiliary of the formula -CH(R')-SH and a γ-mercapto auxiliary of the formula -CH₂-CH(R')-SH,
wherein R' is a residue of an amino acid side chain other than hydrogen and is selected from the group consisting of:
ethylene, phenyl, hydroxyphenyl, allylthioalkyl, carbamoyl (-C(O)NH₂), carbamoylalkyl, indolyl, hydroxycarbonyl, guanidine alkyl, and imidazolyl;
and J₂ is a residue of a second target molecule, polymer or surface.

2. The method of claim 1, further comprising:
removing said thiol auxiliary to generate a ligation product of the formula J₁-C(O)-NH-CH(XH)-J₂, wherein XH is an amino acid side chain having a formula selected from the group consisting of: -CH₂(R') and -CH₂-CH₂(R').

3. The method of claim 2, wherein J₁ and J₂ are the same or different and are selected from the group consisting of a residue of a peptide, a polymer and a surface.

4. The method of claim 1, wherein J₁ is a residue of a peptide.

5. The method of claim 4, wherein -C(O)SR is at the C-terminus of said peptide.

6. The method of claim 1, wherein J₂ is a residue of a peptide.

7. The method of claim 6, wherein NH₂-CH(XSH)- is at the N-terminus of said peptide.

8. The method of claim 7, wherein XSH is the side chain of a β- branched amino acid.

9. The method of claim 8, wherein said β-branched amino acid is phenylcysteine.

10. The method of claim 9, wherein said phenylcysteine is *syn-*phenylcysteine.

11. A component having the formula P₁-NH-CH(XSH)-J₂, wherein P₁ is a removable amino protecting group or hydrogen; XSH is a branched amino acid side chain component and selected from the group consisting of a β-mercapto auxiliary of the formula -CH(R')-SH and a γ-mercapto auxiliary of the formula - CH₂-CH(R')-SH, wherein
R' is other than hydrogen and is selected from the group consisting of: ethylene, phenyl, hydroxyphenyl, allylthioalkyl, carbamoyl (-C(O)NH2), carbamoylalkyl, indolyl, hydroxycarbonyl, aminoalkyl, guanidine alkyl, and imidazolyl,
and the residues CH₂R' and CH₂CH₂R' produced after removal of the thiol auxiliary are side chains of amino acids; and J₂ is a residue of a target molecule, polymer or a surface.

12. The component of claim 11, where J₂ is selected from the group consisting of a residue of a small molecule, a polymer and a surface.

13. The component of claim 11, wherein J₂ is a residue of a peptide.

14. The component of claim 13, wherein P₁-NH-CH(XSH)- is at the N-terminus of said peptide.

15. The component of claim 14, wherein XSH is the side chain of a β-branched amino acid.

16. The component of claim 15, wherein said β-branched amino acid is phenylcysteine.

17. The component of claim 16, wherein said phenylcysteine is *syn*-phenylcysteine.

18. A ligation product having the formula J₁-C(O)-NH-CH(XSH)-J₂, wherein J₁ is a residue of a first ligation component, XSH is a branched amino acid side chain having a removable thiol auxiliary and is selected from the group consisting of a β-mercapto auxiliary of the formula -CH(R')-SH and a γ-mercapto auxiliary of the formula -CH₂-CH(R')-SH, wherein R' is a residue of an amino acid side chain other than hydrogen and is selected from the group consisting of: ethylene, phenyl, hydroxyphenyl, allylthioalkyl, carbamoyl (-C(O)NH2), carbamoylalkyl, indolyl, hydroxycarbonyl, aminoalkyl, guanidine alkyl, and imidazolyl, and J₂ is a residue of a second ligation component.

19. The product of claim 18, where J₁ and J₂ are the same or different and are selected from the group consisting of a residue of a small molecule, a polymer and a surface.

20. The product of claim 18, wherein J₁ is a residue of a peptide.

21. The product of claim 18, wherein J₂ is a residue of a peptide.

22. The product of claim 18, wherein XSH is a β-mercapto auxiliary of the formula -CH(R')-SH, and part of a β-branched amino acid.

23. The product of claim 22, wherein said β-branched amino acid is phenylcysteine.

24. The product of claim 23, wherein said phenylcysteine is *syn*- phenylcysteine.

25. A kit comprising as a component a composition according to claim 11.

## Patentansprüche

1. Ein Verfahren zur Ligation von Komponenten durch eine Amidbindung, wobei das Verfahren Schritte umfasst, bei denen man:
eine Komponente der Formel J₁-C(O)SR mit einer Komponente der Formel NH₂-CH(XSH)-J₂ unter Bedingungen, die zur Bildung eines Thiol-substituierten Ligationsprodukts mit der Formel J₁-C(O)-NH-CH(XSH)-J₂ ausreichend sind, kontaktiert,
wobei
J₁ ein Rest eines ersten Zielmoleküls, Polymers oder einer Oberfläche ist;
R eine mit Thioestern kompatible Gruppe ist;
XSH eine verzweigte Aminosäureseitenkette mit einem entfernbaren Thiol-Auxiliar ist und ausgewählt ist aus der Gruppe bestehend aus einem β-Mercapto-Auxiliar der Formel -CH(R')-SH und einem γ-Mercapto-Auxiliar der Formel -CH₂-CH(R')-SH,
wobei R' ein Rest einer Aminosäureseitenkette außer Wasserstoff ist und ausgewählt ist aus der Gruppe bestehend aus: Ethylen, Phenyl, Hydroxyphenyl, Allylthioalkyl, Carbamoyl (-C(O)NH₂), Carbamoylalkyl, Indolyl, Hydroxycarbonyl, Guanidinalkyl und Imidazolyl;
und J₂ ein Rest eines zweiten Zielmoleküls, Polymers oder einer Oberfläche ist.

2. Das Verfahren nach Anspruch 1, ferner umfassend:
das Entfernen des Thiol- Auxiliars zur Herstellung eines Ligationsprodukts der Formel J₁-C(O)-NH-CH(XH)-J₂, wobei XH eine Aminosäureseitenkette mit einer Formel ausgewählt aus der Gruppe bestehend aus: -CH₂(R') und -CH₂-CH₂(R') ist.

3. Das Verfahren nach Anspruch 2, **dadurch gekennzeichnet, dass** J₁ und J₂ gleich oder unterschiedlich sind und ausgewählt sind aus der Gruppe bestehend aus einem Rest eines Peptids, einem Polymer und einer Oberfläche.

4. Das Verfahren nach Anspruch 1, **dadurch gekennzeichnet, dass** J₁ ein Rest eines Peptids ist.

5. Das Verfahren nach Anspruch 4, **dadurch gekennzeichnet, dass** -C(O)SR sich an dem C-Terminus des Peptids befindet.

6. Das Verfahren nach Anspruch 1, **dadurch gekennzeichnet, dass** J₂ ein Rest eines Peptids ist.

7. Das Verfahren nach Anspruch 6, **dadurch gekennzeichnet, dass** NH₂-CH(XSH)- an dem N-Terminus des Peptids ist.

8. Das Verfahren nach Anspruch 7, **dadurch gekennzeichnet, dass** XSH die Seitenkette einer β-verzweigten Aminosäure ist.

9. Das Verfahren nach Anspruch 8, **dadurch gekennzeichnet, dass** die β-verzweigte Aminosäure Phenylcystein ist.

10. Das Verfahren nach Anspruch 9, **dadurch gekennzeichnet, dass** das Phenylcystein *syn*-Phenylcystein ist.

11. Eine Komponente mit der Formel P₁-NH-CH(XSH)-J₂, wobei P₁ eine entfernbare Aminoschutzgruppe oder Wasserstoff ist; XSH eine verzweigte Aminosäureseitenketten-Komponente ist, und ausgewählt ist aus der Gruppe bestehend aus einem β-Mercapto-Auxiliar der Formel -CH(R')-SH und einem γ-Mercapto-Auxiliar der Formel -CH₂-CH(R')-SH, wobei R' nicht Wasserstoff ist und ausgewählt ist aus der Gruppe bestehend aus: Ethylen, Phenyl, Hydroxyphenyl, Allylthioalkyl, Carbamoyl (-C(O)NH₂), Carbamoylalkyl, Indolyl, Hydroxycarbonyl, Aminoalkyl, Guanidinalkyl und Imidazolyl,
und die Reste CH₂R' und CH₂CH₂R', welche nach Entfernung des Thiol-Auxiliars hergestellt wurden, Seitenketten von Aminosäuren sind; und J₂ ein Rest eines Zielmoleküls, Polymers oder einer Oberfläche ist.

12. Die Komponente nach Anspruch 11, **dadurch gekennzeichnet, dass** J₂ ausgewählt ist aus der Gruppe bestehend aus einem Rest eines kleinen Moleküls, einem Polymer und einer Oberfläche.

13. Die Komponente nach Anspruch 11, **dadurch gekennzeichnet, dass** J₂ ein Rest eines Peptids ist.

14. Die Komponente nach Anspruch 13, **dadurch gekennzeichnet, dass** P₁-NH-CH(XSH)- an dem N-Terminus des Peptids ist.

15. Die Komponente nach Anspruch 14, **dadurch gekennzeichnet, dass** XSH die Seitenkette einer β-verzweigten Aminosäure ist.

16. Die Komponente nach Anspruch 15, **dadurch gekennzeichnet, dass** die β-verzweigte Aminosäure Phenylcystein ist.

17. Die Komponente nach Anspruch 16, **dadurch gekennzeichnet, dass** das Phenylcystein syn-Phenylcystein ist.

18. Ein Ligationsprodukt mit der Formel J₁-C(O)-NH-CH(XSH₎-J₂, wobei J₁ ein Rest einer ersten Ligationskomponente ist, XSH eine verzweigte Aminosäureseitenkette mit einem entfernbaren Thiol-Auxiliar ist und ausgewählt ist aus der Gruppe bestehend aus einem β-Mercapto-Auxiliar der Formel -CH(R')-SH und einem γ-Mercapto-Auxiliar der Formel -CH₂-CH(R')-SH, wobei R' ein Rest einer Aminosäureseitenkette außer Wasserstoff ist und ausgewählt ist aus der Gruppe bestehend aus: Ethylen, Phenyl, Hydroxyphenyl, Allylthioalkyl, Carbamoyl (-C(O)NH₂), Carbamoylalkyl, Indolyl, Hydroxycarbonyl, Aminoalkyl, Guanidinalkyl und Imidazolyl, und J₂ ein Rest einer zweiten Ligationskomponente ist.

19. Das Produkt nach Anspruch 18, **dadurch gekennzeichnet, dass** J₁ und J₂ gleich oder verschieden sind und ausgewählt sind aus der Gruppe bestehend aus einem Rest eines kleinen Moleküls, einem Polymers und einer Oberfläche.

20. Das Produkt nach Anspruch 18, **dadurch gekennzeichnet, dass** J₁ ein Rest eines Peptids ist.

21. Das Produkt nach Anspruch 18, **dadurch gekennzeichnet, dass** J₂ ein Rest eines Peptids ist.

22. Das Produkt nach Anspruch 18, **dadurch gekennzeichnet, dass** XSH ein β-Mercapto-Auxiliar der Formel -CH(R')-SH ist und Teil einer β-verzweigten Aminosäure ist.

23. Das Produkt nach Anspruch 22, **dadurch gekennzeichnet, dass** die β-verzweigte Aminosäure Phenylcystein ist.

24. Das Produkt nach Anspruch 23, **dadurch gekennzeichnet, dass** das Phenylcystein *syn*-Phenylcystein ist.

25. Ein Kit, umfassend als eine Komponente eine Zusammensetzung nach Anspruch 11.

## Revendications

1. Procédé de liaison de composants par l'intermédiaire d'une liaison de type amide, lequel procédé comporte :
le fait de mettre un composant de formule J₁-C(O)SR en contact avec un composant de formule NH₂-CH(XSH)-J₂, dans des conditions appropriées pour qu'il se forme un produit de liaison porteur d'un substituant à fonction thiol, de formule J₁-C(O)-NH-CH(XSH)-J₂,
dans lesquelles formules :
- J₁ représente un résidu d'une première cible, qui est une molécule, un polymère ou une surface ;
- R représente un groupe compatible avec les thioesters ;
- XSH représente une chaîne latérale d'un acide aminé ramifié, comportant un groupe auxiliaire sulfhydryle amovible et choisie parmi une chaîne à groupe auxiliaire β-sulfhydryle, de formule -CH(R')-SH, et une chaîne à groupe auxiliaire γ-sulfhydryle, de formule -CH₂-CH(R')-SH, où R' représente un reste d'une chaîne latérale d'acide aminé, autre qu'un atome d'hydrogène et choisi dans l'ensemble formé par les groupes éthylène, phényle, hydroxyphényle, allylthio-alkyle, carbamyle -C(O)NH₂, carbamyl-alkyle, indolyle, hydroxy-carbonyle, guanidino-alkyle et imidazolyle ;
- et J₂ représente un résidu d'une deuxième cible, qui est une molécule, un polymère ou une surface.

2. Procédé conforme à la revendication 1, qui comporte en outre le fait d'éliminer ledit groupe auxiliaire sulfhydryle de manière à ce qu'il se forme un produit de liaison de formule J₁-C(O)-NH-CH(XH)-J₂, dans laquelle XH représente une chaîne latérale d'acide aminé de formule choisie parmi -CH₂(R') et -CH₂-CH₂(R').

3. Procédé conforme à la revendication 2, dans lequel J₁ et J₂ représentent des entités identiques ou différentes, qui sont choisies dans l'ensemble formé par les résidus d'un peptide, d'un polymère ou d'une surface.

4. Procédé conforme à la revendication 1, dans lequel J₁ représente le résidu d'un peptide.

5. Procédé conforme à la revendication 4, dans lequel le groupe de formule -C(O)SR se situe à l'extrémité carboxy-terminale dudit peptide.

6. Procédé conforme à la revendication 1, dans lequel J₂ représente le résidu d'un peptide.

7. Procédé conforme à la revendication 6, dans lequel le groupe de formule NH₂-CH(XSH)- se situe à l'extrémité amino-terminale dudit peptide.

8. Procédé conforme à la revendication 7, dans lequel le groupe de formule XSH est la chaîne latérale d'un acide aminé β-ramifié.

9. Procédé conforme à la revendication 8, dans lequel ledit acide aminé β-ramifié est une phényl-cystéine.

10. Procédé conforme à la revendication 9, dans lequel ladite phényl-cystéine est la *syn*-phényl-cystéine.

11. Composant de formule P₁-NH-CH(XSH)-J₂, dans laquelle :
- P₁ représente un groupe amino-protecteur amovible ou un atome d'hydrogène ;
- XSH représente une chaîne latérale d'un acide aminé ramifié, qui est choisie parmi une chaîne à groupe auxiliaire β-sulfhydryle, de formule -CH(R')-SH, et une chaîne à groupe auxiliaire γ-sulfhydryle, de formule -CH₂-CH(R')-SH,
où R' représente une entité autre qu'un atome d'hydrogène et choisie dans l'ensemble constitué par les groupes éthylène, phényle, hydroxyphényle, allylthio-alkyle, carbamyle -C(O)NH₂, carbamyl-alkyle, indolyle, hydroxy-carbonyle, amino-alkyle, guanidino-alkyle et imidazolyle, les résidus de formule CH₂R' et CH₂CH₂R' formés après élimination du groupe sulfhydryle auxiliaire étant des chaînes latérales d'acides aminés ;
- et J₂ représente le résidu d'une cible, qui est une molécule, un polymère ou une surface.

12. Composant conforme à la revendication 11, dans lequel J₂ représente une entité choisie parmi des résidus d'une petite molécule, d'un polymère ou d'une surface.

13. Composant conforme à la revendication 11, dans lequel J₂ représente le résidu d'un peptide.

14. Composant conforme à la revendication 13, dans lequel le groupe de formule P₁-NH-CH(XSH)- se situe à l'extrémité amino-terminale dudit peptide.

15. Composant conforme à la revendication 14, dans lequel le groupe de formule XSH est la chaîne latérale d'un acide aminé β-ramifié.

16. Composant conforme à la revendication 15, dans lequel ledit acide aminé β-ramifié est une phényl-cystéine.

17. Composant conforme à la revendication 16, dans lequel ladite phényl-cystéine est la *syn*-phényl-cystéine.

18. Produit de liaison de formule J₁-C(O)-NH-CH(XSH)-J₂, dans laquelle :
- J₁ représente le résidu d'un premier partenaire de liaison ;
- XSH représente une chaîne latérale d'un acide aminé ramifié, qui est choisie parmi une chaîne à groupe auxiliaire β-sulfhydryle, de formule -CH(R')-SH, et une chaîne à groupe auxiliaire γ-sulfhydryle, de formule -CH₂-CH(R')-SH,
où R' représente le reste d'une chaîne latérale d'acide aminé, autre qu'un atome d'hydrogène et choisi dans l'ensemble constitué par les groupes éthylène, phényle, hydroxyphényle, allylthio-alkyle, carbamyle -C(O)NH₂, carbamyl-alkyle, indolyle, hydroxy-carbonyle, amino-alkyle, guanidino-alkyle et imidazolyle ;
- et J₂ représente le résidu d'un deuxième partenaire de liaison.

19. Produit conforme à la revendication 18, dans lequel J₁ et J₂ représentent des entités identiques ou différentes, qui sont choisies dans l'ensemble formé par les résidus d'une petite molécule, d'un polymère ou d'une surface.

20. Produit conforme à la revendication 18, dans lequel J₁ représente le résidu d'un peptide.

21. Produit conforme à la revendication 18, dans lequel J₂ représente le résidu d'un peptide.

22. Produit conforme à la revendication 18, dans lequel le groupe de formule XSH est une chaîne à groupe auxiliaire β-sulfhydryle, de formule -CH(R')-SH, et fait partie d'un acide aminé β-ramifié.

23. Produit conforme à la revendication 22, dans lequel ledit acide aminé β-ramifié est une phényl-cystéine.

24. Produit conforme à la revendication 23, dans lequel ladite phényl-cystéine est la *syn*-phényl-cystéine.

25. Trousse comprenant comme composant un composant conforme à la revendication 11.
